# EUROPÄISCHE PATENTSCHRIFT

(11) **EP 1 478 610 B1**
(45) Veröffentlichungstag und Bekanntmachung des Hinweises auf die Patenterteilung: **07.01.2009**
(21) Anmeldenummer: 03709709.4
(22) Anmeldetag: 17.02.2003
(51) Int. Cl.: C07C 2/52, C07C 5/367

(54) **VERFAHREN ZUR HERSTELLUNG VON 4-VINYLCYCLOHEXEN, ETHYLBENZOL UND STYROL**
METHOD FOR PRODUCING 4-VINYLCYCLOHEXENE, ETHYL BENZOLE AND STYRENE
PROCEDE DE PRODUCTION DE 4-VINYLCYCLOHEXENE, ETHYLBENZENE ET STYRENE

(30) Priorität: 19.02.2002 DE 10206954; 12.07.2002 DE 10231633
(43) Veröffentlichungstag der Anmeldung: 24.11.2004
(73) Patentinhaber: BASF SE, 67056 Ludwigshafen (DE)
(72) Erfinder: WALSDORFF, Christian, 67059 Ludwigshafen (DE); SCHINDLER, Götz-Peter, 68219 Mannheim (DE); HARTH, Klaus, 67317 Altleiningen (DE); HIBST, Hartmut, 69198 Schriesheim (DE)
(74) Vertreter: Isenbruck, Günter
(86) Internationale Anmeldenummer: PCT/EP2003/001577
(87) Internationale Veröffentlichungsnummer: WO 2003/070671

(56) Entgegenhaltungen:
- WO-A-94/01385
- US-A- 2 376 985
- US-A- 2 438 041
- US-A- 3 502 736
- US-A- 4 029 715
- US-A- 5 196 621
- US-A- 5 321 180

## Beschreibung

Die Erfindung betrifft Verfahren zur Herstellung von 4-Vinylcyclohexen sowie von Ethylbenzol und von Styrol als Folgeprodukte des 4-Vinylcyclohexens.

Es ist bekannt, 4-Vinylcyclohexen durch Cyclodimerisierung von 1,3-Butadien in flüssiger Phase in Gegenwart von Cu(I)-Ionen enthaltenden Trägerkatalysatoren herzustellen. Das gebildete 4-Vinylcyclohexen kann in einer nachgeschalteten Dehydrierstufe zu Ethylbenzol dehydriert oder in Gegenwart von Sauerstoff direkt zu Styrol oxidehydriert werden.

US 5,196,621 offenbart ein Verfahren zur Butadien-Dimerisierung in flüssiger Phase an mit Cu(I)-Ionen imprägnierten Aluminosilicaten als Dimerisierungskatalysatoren, vorzugsweise an mit Cu(I)-Ionen imprägnierten Zeolithen wie Faujasit, Mordenit, Zeolith L, Zeolith omega und Zeolith beta. Ferner werden Cu(I)-Ionen enthaltende Tonmineralien wie Montmorillonit und Cu(I)-Ionen enthaltende nicht-zeolithische amorphe Aluminiumoxid/Siliziumdioxid-Mischungen, Siliziumdioxid oder Aluminiumoxid als geeignete Katalysatoren genannt.

Butadien wird überwiegend durch thermische Spaltung (Cracken) gesättigter Kohlenwasserstoffe hergestellt, wobei üblicherweise von Naphtha als Rohstoff ausgegangen wird. Beim Cracken von Naphtha fällt ein Kohlenwasserstoff-Gemisch aus Methan, Ethan, Ethen, Acetylen, Propan, Propen, Propin, Allen, Butenen, Butadien, Butinen, Methylallen, C₅- und höheren Kohlenwasserstoffen an. Störend bei der Dimerisierung sind insbesondere acetylenisch ungesättigte Kohlenwasserstoffe im Crackgas wie Acetylen, Propin, 1-Butin, 2-Butin, Butenin und Diacetylen. Schon Spuren dieser Verbindungen können den kupferhaltigen Dimerisierungskatalysator vergiften. Butine und Allene reagieren ebenfalls in einer Diels-Alder-Reaktion mit Butadien und führen zu Nebenproduktbildung. Probleme bereiten insbesondere die Butine, die sich von Butadien nur sehr schwer destillativ oder extraktiv abtrennen lassen. Daher ist es bei der Verwendung von Butadien aus Crackern erforderlich, der Butadien-Dimerisierung eine Hydrierstufe vorzuschalten, in der die Butine selektiv partiell zu den entsprechenden Butenen hydriert werden. Auch bei anderen Verwendungen von Butadien sind dreifach ungesättigte C₄-Kohlenwasserstoffe in der Regel störend.

Nachteilig ist ferner, dass beim Cracken von Naphtha oder anderen KohlenwasserstoffGemischen ein komplexes Kohlenwasserstoff-Gemisch erzeugt wird. So fallen bei der Erzeugung von Butadien im Crackprozess zwangsläufig größere Mengen an Ethen oder Propen als Koppelprodukte an.

Aufgabe der Erfindung ist es, ein wirtschaftliches Verfahren zur Herstellung von 4-Vinylcyclohexen, Ethylbenzol bzw. Styrol bereitzustellen, bei dem weniger Koppelprodukte anfallen. Aufgabe der Erfindung ist es insbesondere, die Herstellung von 4-Vinylcyclohexen, Ethylbenzol und Styrol auf eine neue Rohstoffbasis zu stellen.

Gelöst wird die Aufgabe durch ein Verfahren zur Herstellung von 4-Vinylcylohexen mit den Schritten
(A) Bereitstellung eines n-Butan enthaltenden Einsatzgastroms,
(B) Einspeisung des n-Butan enthaltenden Einsatzgasstroms in mindestens eine Dehydrierzone und Dehydrierung von n-Butan zu Butadien, wobei ein Butadien, n-Butan, gegebenenfalls 1-Buten und 2-Buten und gegebenenfalls Wasserdampf und weitere Nebenbestandteile enthaltender Produktstrom erhalten wird,
(C) Einspeisung des Produktstroms der Dehydrierung, gegebenenfalls nach Abtrennung von Wasserdampf und Nebenbestandteilen, in eine Dimerisierungszone und katalytische Dimerisierung von Butadien, wobei ein 4-Vinylcyclohexen, n-Butan und gegebenenfalls 1-Buten, 2-Buten und nicht umgesetztes Butadien enthaltender Produktstrom erhalten wird,
(D) Abtrennung von 4-Vinylcyclohexen aus dem Produktstrom der Dimerisierung und Rückführung von n-Butan und gegebenenfalls 1-Buten, 2-Buten und nicht umgesetztem Butadien in die Dehydrierzone.

Bei der Dehydrierung von n-Butan werden keine acetylenisch ungesättigten Kohlenwasserstoffe oder Allene in wesentlichen Mengen als Nebenprodukte gebildet. Somit kann eine partielle Hydrierung des bei der Butadien-Dimerisierung eingesetzten Gasgemischs entfallen.

In einem ersten Verfahrensteil A wird ein n-Butan enthaltender Einsatzgasstrom bereitgestellt. Üblicherweise wird dabei von an n-Butan reichen Gasgemischen wie liquefied petroleum gas (LPG) als Rohstoff ausgegangen. LPG enthält im wesentlichen C₂-C₅-Kohlenwasserstoffe. Die Zusammensetzung von LPG kann stark schwanken. Vorteilhafter Weise enthält das eingesetzte LPG mindestens 10 Gew.-% Butane.

In einer Variante des erfindungsgemäßen Verfahrens umfasst die Bereitstellung des n-Butan enthaltenden Dehydrier-Einsatzstromes die Schritte
(A1) Bereitstellung eines liquefied petroleum gas (LPG)-Stroms,
(A2) Abtrennung von Propan und gegebenenfalls Methan, Ethan und Pentanen aus dem LPG-Strom, wobei ein Butane enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

Die Abtrennung von Propan und gegebenenfalls Methan, Ethan und Pentanen erfolgt in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (Pentane) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom wird vorzugsweise einer Isomerisierung unterworfen. Dazu wird der Isobutan enthaltende Strom in einen Isomerisierungsreaktor eingespeist. Die Isomerisierung von Isobutan zu n-Butan kann wie in GB-A 2 018 815 beschrieben durchgeführt werden. Es wird ein n-Butan/Isobutan-Gemisch erhalten, das in die n-Butan/Isobutan-Trennkolonne eingespeist wird.

In einem Verfahrensteil (B) wird n-Butan zu Butadien dehydriert.

In einer Ausführungsform des erfindungsgemäßen Verfahrens wird die Butan-Dehydrierung als nicht-oxidative katalytische Dehydrierung durchgeführt. Dabei wird n-Butan in einem Dehydrierreaktor an einem dehydrieraktiven Katalysator teilweise zu Butadien dehydriert. Daneben werden aus n-Butan 1-Buten und 2-Buten gebildet. Bei der Dehydrierung fallen neben Wasserstoff in geringen Mengen Methan, Ethan, Ethen, Propan und Propen an. Je nach Fahrweise der Dehydrierung können außerdem Kohlenstoffoxide (CO, CO₂), Wasser und Stickstoff im Produktgasgemisch der Butan-Dehydrierung enthalten sein. Daneben liegt im Produktgasgemisch nicht umgesetztes n-Butan vor.

Die nicht-oxidative katalytische Butan-Dehydrierung kann mit oder ohne sauerstoffhaltigem Gas als Co-Feed durchgeführt werden.

Die nicht-oxidative katalytische n-Butan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung (analog dem Linde-Verfahren zur Propan-Dehydrierung), aber auch zwischen 3 und 8 bar bei Verwendung einer hohen Wasserdampfverdünnung (analog dem sogenannten "steam active reforming process" (STAR-Prozess) zur Dehydrierung von Propan oder Butan von Phillips Petroleum Co., siehe US 4,902,849, US 4,996,387 und US 5,389,342). Typische Katalysatorbelastungen (GHSV) liegen bei 500 bis 2000 h⁻¹, bezogen auf den eingesetzten Kohlenwasserstoff. Die Katalysatorgeometrie kann beispielsweise kugelförmig oder zylindrisch (hohl oder voll) sein.

Die nicht-oxidative katalytische n-Butan-Dehydrierung kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff in Gegenwart von Sauerstoff erzeugt werden. Gegebenenfalls kann zusätzlich ein Wasserstoff enthaltender Co-Feed zugemischt werden.

Die nicht-oxidative katalytische n-Butan-Dehydrierung kann mit oder ohne sauerstoffhaltigem Gas als Co-Feed in einem Hordenreaktor durchgeführt werden. Dieser enthält ein oder mehrere aufeinanderfolgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform, wobei mit sauerstoffhaltigem Co-Feed gearbeitet werden kann. In einer weiteren bevorzugten Ausführungsform wird die Dehydrierung in einem Hordenreaktor mit 3 Katalysatorbetten durchgeführt. Bei einer Fahrweise ohne sauerstoffhaltigem Gas als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherflächen oder durch Durchleiten durch mit heißen Brenngasen beheizte Rohre.

In einer bevorzugten Ausführungsform des erfindungsgemäßen Verfahrens wird die nicht-oxidative katalytische n-Butan-Dehydrierung autotherm durchgeführt. Dazu wird dem Reaktionsgasgemisch der n-Butan-Dehydrierung in mindestens einer Reaktionszone zusätzlich Sauerstoff zugemischt und der in dem Reaktionsgasgemisch enthaltene Wasserstoff und/oder Kohlenwasserstoffe zumindest teilweise verbrannt, wodurch zumindest ein Teil der benötigten Dehydrierwärme in der mindestens einen Reaktionszone direkt in dem Reaktionsgasgemisch erzeugt wird. Ein Merkmal der autothermen Fahrweise gegenüber einer oxidativen Fahrweise ist beispielsweise das Vorhandensein von Wasserstoff im Austragsgas. Bei den sogenannten oxidativen Verfahren wird kein freier Wasserstoff in wesentlichen Mengen gebildet.

Im allgemeinen wird die Menge des dem Reaktionsgasgemisch zugesetzten sauerstoffhaltigen Gases so gewählt, dass durch die Verbrennung von im Reaktionsgasgemisch vorhandenem Wasserstoff und gegebenenfalls von im Reaktionsgasgemisch vorliegenden Kohlenwasserstoffen und/oder von in Form von Koks vorliegendem Kohlenstoff die für die Dehydrierung des n-Butans benötigte Wärmemenge erzeugt wird. Im allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol. Sauerstoff kann entweder als reiner Sauerstoff oder als sauerstoffhaltiges Gas im Gemisch mit Inertgasen, beispielsweise in Form von Luft, eingesetzt werden. Die Inertgase und die resultierenden Verbrennungsgase wirken im allgemeinen zusätzlich verdünnend und fördern damit die heterogen katalysierte Dehydrierung.

Der zur Wärmeerzeugung verbrannte Wasserstoff ist der bei der katalytischen n-Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stellen des Reaktors erfolgen.

In einer Ausführungsform des erfindungsgemäßen Verfahrens erfolgt eine Zwischeneinspeisung von sauerstoffhaltigem Gas und von wasserstofthaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen n-Butan-Dehydrierung sind die Katalysatoren gemäß den Beispielen 1, 2, 3 und 4 der DE-A 199 37 107.

Die n-Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid und Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Die Gegenwart von verdünnend wirkendem Wasserdampf wirkt sich auch positiv auf die Lage des chemischen Gleichgewichts aus, das auf die Seite der Produkte der Dehydrierung verschoben wird. Gegebenenfalls wir nach Durchführung der Regenerierung mit Wasserdampf noch mit einem wasserstoffhaltigen Gas reduziert.

Bei der Butan-Dehydrierung wird ein Gasgemisch erhalten, das neben Butadien, 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die Dehydrierstufe verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen Butan-Dehydrierung enthält typischerweise 0,1 bis 15 Vol.-% Butadien, 1 bis 15 Vol.-% 1-Buten, 1 bis 20 Vol.-% 2-Buten, 20 bis 70 Vol.-% Butan, 5 bis 70 Vol.-% Wasserdampf, 0 bis 5 Vol.-% leichtsiedene Kohlenwasserstoffe (Methan, Ethan, Ethen, Propan und Propen), 0 bis 30 Vol.-% Wasserstoff, 0 bis 30 Vol.-% Stickstoff und 0 bis 5 Vol.-% Kohlenstoffoxide.

In einer bevorzugten Ausführungsform wird der nicht-oxidativen katalytischen Dehydrierung eine oxidative Dehydrierung nachgeschaltet.

Die Dehydrierung von n-Butan zu Butadien umfasst nach dieser bevorzugten Ausführungsform die Schritte
(B1) Einspeisung des n-Butan enthaltenden Einsatzgasstroms in eine erste Dehydrierzone und katalytische, nicht-oxidative Dehydrierung von n-Butan zu 1-Buten, 2-Buten und gegebenenfalls Butadien, wobei ein n-Butan, 1-Buten, 2-Buten, gegebenenfalls Butadien und gegebenenfalls Nebenbestandteile enthaltender Produktgasstrom erhalten wird,
(B2) Einspeisung des n-Butan, 1-Buten, 2-Buten, gegebenenfalls Butadien und gegebenenfalls Nebenbestandteile enthaltenden Produktgasstroms in eine zweite Dehydrierzone und oxidative Dehydrierung von 1-Buten und 2-Buten zu Butadien, wobei ein Butadien, n-Butan, Wasserdampf und gegebenenfalls Nebenbestandteile enthaltender Produktgasstrom erhalten wird.

Bevorzugt wird die katalytische nicht-oxidative Dehydrierung (B1) von n-Butan zu 1-Buten, 2-Buten und gegebenenfalls Butadien wie oben beschrieben als autotherme Dehydrierung durchgeführt.

Die Oxidehydrierung (B2) kann grundsätzlich in allen aus dem Stand der Technik bekannten Reaktortypen und Fahrweisen durchgeführt werden, wie beispielsweise im Wirbelbett, im Hordenofen oder im Festbettrohr- oder -rohrbündelreaktor. Letzterer wird bei dem erfindungsgemäßen Verfahren bevorzugt eingesetzt. Zur Durchführung der oxidativen Dehydrierung wird ein Gasgemisch benötigt, welches ein molares Sauerstoff : n-Butene-Verhältnis von mindestens 0,5 aufweist. Bevorzugt wird bei einem Sauerstoff : n-Butene-Verhältnis von 0,55 bis 50 gearbeitet. Zur Einstellung dieses Wertes wird in der Regel das aus der katalytischen Dehydrierung stammende Produktgasgemisch mit Sauerstoff oder einem sauerstoffhaltigem Gas, beispielsweise Luft, vermischt. Das erhaltene sauerstoffhaltige Gasgemisch wird dann der Oxidehydrierung zugeführt.

Die für die oxidative Dehydrierung (Oxidehydrierung) der n-Butene zu 1,3-Butadien besonders geeigneten Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan, Mangan, Wolfram, Phosphor, Cer, Aluminium oder Silizium.

Geeignete Katalysatoren und deren Herstellung sind beispielsweise beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ), worauf explizit Bezug genommen wird.

Die Stöchiometrie der Aktivmasse einer Vielzahl der für die Oxidehydrierung der n-Butene zu 1,3-Butadien geeigneten Multimetalloxidkatalysatoren lässt sich unter die allgemeine Formel (I)

Mo₁₂BiₐFe_{b}Co_{c}Ni_{d}CrₑX¹_{f}K_{g}Oₓ (I),

in der die Variablen nachfolgende Bedeutung aufweisen:
- X¹ =: W, Sn, Mn, La, Ce, Ge, Ti, Zr, Hf, Nb, P, Si, Sb, Al, Cd und/oder Mg;
- a =: 0,5 bis 5, vorzugsweise 0,5 bis 2;
- b =: 0 bis 5, vorzugsweise 2 bis 4;
- c =: 0 bis 10, vorzugsweise 3 bis 10;
- d =: 0 bis 10;
- e =: 0 bis 10, vorzugsweise 0,1 bis 4;
- f =: 0 bis 5, vorzugsweise 0,1 bis 2;
- g =: 0 bis 2, vorzugsweise 0,01 bis 1; und
- x =: eine Zahl, die durch die Wertigkeit und Häufigkeit der von Sauerstoff verschiedenen Elemente in (I) bestimmt wird;
subsumieren.

Bevorzugt setzt man beim erfindungsgemäßen Verfahren für die Oxidehydrierung ein Mo-Bi-Fe-O-haltiges Multimetalloxidsystem ein, wobei ein Mo-Bi-Fe-Cr-O- oder Mo-Bi-Fe-Zr-O-haltiges Multimetalloxidsystem besonders bevorzugt ist. Bevorzugte Systeme sind beispielsweise beschrieben in US 4,547,615 (Mo₁₂BiFe_{0,1}Ni₈ZrCr₃K_{0,2}Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A 25 30 959 (Mo₁₂BiFe₃Co_{4,5}Ni_{2,5}Cr_{0,5}K_{0,1}Oₓ, Mo_{13,75}BiFe₃Co_{4,5}Ni_{2,5}Ge_{0,5}K_{0,8}Oₓ, Mo₁₂BiFe₃Co_{4,5}Ni_{2,5}Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ). Herstellung und Charakterisierung der genannten Katalysatoren sind ausführlich in den zitierten Schriften beschrieben, auf die hiermit explizit verwiesen wird.

Der Katalysator zur Oxidehydrierung wird im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise genannt Tabletten, Zylinder, Hohlzylinder, Ringe, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen kann der verwendete Katalysator als sogenannter Vollkatalysator eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Insbesondere hat es sich als günstig erwiesen, den für die Oxidehydrierung der n-Butene zu Butadien bevorzugt eingesetzten Mo-Bi-Fe-O-haltigen Katalysator als Vollkatalysator einzusetzen. Des weiteren ist es möglich, die Aktivmassen der Katalysatoren auf einen Träger, beispielsweise einen anorganischen, oxidischen Formkörper, aufzubringen. Derartige Katalysatoren werden in der Regel als Schalenkatalysatoren bezeichnet.

Die Oxidehydrierung der n-Butene zu Butadien wird im Allgemeinen bei einer Temperatur von 220 bis 490 °C und bevorzugt von 250 bis 450 °C durchgeführt. Aus praktischen Erwägungen wählt man in der Regel einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung an Katalysatoren und inerten Anteilen ab.

Durch die Koppelung der nicht-oxidativen katalytischen, bevorzugt autothermen Dehydrierung mit der oxidativen Dehydrierung der gebildeten n-Butene wird eine sehr viel höhrere Ausbeute an Butadien, bezogen auf eingesetztes n-Butan, erhalten. Ferner kann die nicht-oxidative Dehydrierung schonender betrieben werden. Vergleichbare Ausbeuten an Butadien wären mit einer ausschließlich nicht-oxidativen Dehydrierung nur um den Preis deutlich niedrigerer Selektivitäten zu erreichen.

Der die oxidative Dehydrierung verlassende Produktgasstrom enthält neben Butadien und nicht umgesetztem n-Butan Wasserdampf. Als Nebenbestandteile enthält er im allgemeinen Kohlenmonoxid, Kohlendioxid, Stickstoff, Sauerstoff, Methan, Ethan, Ethen, Propan und Propen, gegebenenfalls Wasserstoff sowie sauerstoffhaltige Kohlenwasserstoffe (Oxygenate). Im allgemeinen enthält er nur noch geringe Anteile an 1-Buten und 2-Buten.

Beispielsweise kann der die oxidative Dehydrierung verlassende Produktgasstrom 1 bis 20 Vol.-% Butadien, 0 bis 1 Vol.-% 1-Buten, 0 bis 1 Vol.-% 2-Buten, 0 bis 50 Vol.-% n-Butan, 2 bis 50 Vol.-% Wasserdampf, 0 bis 5 Vol.-% leichtsiedene Kohlenwasserstoffe (Methan, Ethan, Ethen, Propan und Propen), 0 bis 20 Vol.-% Wasserstoff, 0 bis 90 Vol.-% Stickstoff, 0 bis 5 Vol.-% Kohlenstoffoxide und 0 bis 3 Gew.-% Oxygenate enthalten.

Nach Verlassen der Dehydrierstufe(n) wird das heiße Gasgemisch, dessen Temperatur bei alleiniger Durchführung der autothermen Dehydrierung in der Regel von 500 bis 650 °C beträgt und bei Durchführung der autothermen Dehydrierung mit nachgeschalteter oxidativer Dehydrierung in der Regel von 220 bis 490 °C beträgt, üblicherweise mit Wasser abgekühlt. Dabei werden Wasserdampf und gegebenenfalls hochsiedende organische Nebenbestandteile auskondensiert. Die in dem Dehydriergasgemisch neben Butadien, n-Butan und gegebenenfalls 1-Buten und 2-Buten enthaltenen leichtsiedenden Nebenbestandteile wie Wasserstoff, Kohlenmonoxid, Kohlendioxid, Stickstoff, Methan, Ethan, Ethen, Propan und Propen werden üblicherweise vor der Butadien-Dimerisierung von den C₄-Kohlenwasserstoffen abgetrennt.

Die Abtrennung der leichtsiedenden Nebenbestandteile kann durch übliche Rektifikation erfolgen.

Die Abtrennung der leichtsiedenden Nebenbestandteile kann auch in einem Absorptions/Desorptions-Cyclus mittels eines hochsiedenden Absorptionsmittels erfolgen. Auf diese Weise werden im wesentlichen alle leichtsiedenden Nebenbestandteile (Stickstoff, Argon, Wasserstoff, Methan, Ethan, Ethen Propan, Propen, Kohlenstoffoxide, Sauerstoff) aus dem Produktgasstrom der n-Butan-Dehydrierung abgetrennt.

Dazu werden in einer Absorptionsstufe die C₄-Kohlenwasserstoffe in einem inerten Absorptionsmittel absorbiert, wobei ein mit den C₄-Kohlenwasserstoffen beladenes Absorptionsmittel und ein die übrigen Nebenbestandteile enthaltendes Abgas erhalten werden. In einer Desorptionsstufe werden die C₄-Kohlenwasserstoff und Spuren von Nebenbestandteilen aus dem Absorptionsmittel wieder freigesetzt.

In der Absorptionsstufe eingesetzte inerte Absorptionsmittel sind im allgemeinen hochsiedende unpolare Lösungsmittel, in denen das abzutrennende Kohlenwasserstoff-Gemisch eine deutlich höhere Löslichkeit als die übrigen Bestandteile der Produktgasmischung aufweisen. Die Absorption kann durch einfaches Durchleiten der Produktgasmischung durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Geeignete Absorptionskolonnen sind z.B. Bodenkolonnen mit Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak^{®} 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

Geeignete Absorptionsmittel sind vergleichsweise unpolare organische Lösungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%. Geeignete Absorptionsmittel sind ferner Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption wird das beladene Absorptionsmittel erhitzt und/oder auf einen niedrigeren Druck entspannt. Alternativ dazu kann die Desorption auch durch Strippung oder in einer Kombination von Entspannung, Erhitzen und Strippung in einem oder mehreren Verfahrensschritten erfolgen. Das in der Desorptionsstufe regenerierte Absorptionsmittel wird in die Absorptionsstufe zurückgeführt.

Aus dem verbleibenden, die C₄-Kohlenwasserstoffe enthaltenden Strom können in einer weiteren Trennstufe, die ebenfalls als Absorptions/Desorptions-Cyclus oder als Rektifikation ausgestaltet sein kann, Oxygenate abgetrennt werden. Oxygenate sind beispielsweise Furan und Maleinsäureanhydrid.

Der verbleibende, im wesentlichen aus Butadien, n-Butan, 1-Buten und 2-Buten bestehende Strom wird der Dimerisierung zugeführt.

In einem sich anschließenden Verfahrensteil (C) wird Butadien katalytisch zu 4-Vinylcyclohexen dimerisiert.

Die Butadien-Dimerisierung kann in flüssiger Phase an einem Kupfer enthaltenden Katalysator durchgeführt werden. Geeignete Dimerisierungskatalysatoren sind mit Cu(I)-Ionen imprägnierte Aluminosilicate, beispielsweise mit Cu(I)-Ionen imprägnierte Zeolithe wie Faujasit, Mordenit, Zeolith L, Zeolith omega und Zeolith beta, wie sie in US 5,196,621 beschrieben sind. Geeignete Träger sind auch Tonmineralien wie Montmorillonit, nicht-zeolithische amorphe Aluminiumoxid/Siliziumdioxid-Mischungen, Siliziumdioxid oder Aluminiumoxid.

Die Butadien-Dimerisierung kann in allen üblichen Reaktionsapparaten in Festbett- oder Suspensionsfahrweise durchgeführt werden, beispielsweise in Rohrreaktoren, kontinuierlich betriebenen Rührkesseln oder Rührkesselkaskaden. Die Reaktionstemperatur beträgt typischerweise von 70 bis 170 °C, vorzugsweise von 100 bis 130 °C, der Reaktionsdruck von 7 bis 70 bar, vorzugsweise von 20 bis 35 bar.

Bei der Butadien-Dimerisierung wird mit hoher Selektivität 4-Vinylcyclohexen gebildet. 1-Buten und 2-Buten, aber auch gegebenenfalls in Spuren vorhandenes Propen, reagieren unter den Reaktionsbedingungen im allgemeinen nicht, da sie über keine für die Diels-Alder-Reaktion aktivierte Doppelbindung verfügen.

Die Butadien-Dimerisierung kann auch, wie in EP-A 0 397 266 beschrieben, in flüssiger Phase in einem geeigneten Lösungsmittel mit Eisen-, Kobalt- oder Nickelnitrosylchlorid in Gegenwart von Kohlenmonoxid und Zinn, Zink, Mangan und/oder Magnesium durchgeführt werden. Geeignete Lösungsmittel sind beispielsweise Ethylenglykol- oder Diethylenglykoldialkylether, Tetrahydrofuran oder Acetonitril. Die Rektionstemperatur beträgt im allgemeinen von 20 bis 175 °C, der Reaktionsdruck von 1 bis 70 bar. Gebildetes Vinylcyclohexen kann anschließend von dem Lösungsmittel destillativ abgetrennt werden.

In einem weiteren Verfahrensteil (D) wird aus dem Austragsstrom der Butadien-Dimerisierung 4-Vinylcyclohexen abgetrennt. Die Abtrennung kann in einer üblichen Rektifizierkolonne erfolgen. Dabei wird im allgemeinen ein Strom aus rohem 4-Vinylcyclohexen, das in geringen Mengen C₈-Nebenprodukte enthalten kann, und ein C₄-Strom, enthaltend n-Butan, 1-Buten, 2-Buten und nicht umgesetztes Butadien, gewonnen. Der C₄-Strom kann in die n-Butan-Dehydrierung zurückgeführt werden.

Das gewonnene 4-Vinylcyclohexen kann, gegebenenfalls nach vorheriger Aufreinigung, anschließend zu Ethylbenzol dehydriert oder aber in Gegenwart von Sauerstoff zu Styrol oxidehydriert werden.

Gegenstand der vorliegenden Erfindung ist somit auch ein Verfahren zur Herstellung von Ethylbenzol oder Styrol mit den Schritten (A), (B), (C) und (D), wie obenstehend beschrieben, und dem zusätzlichen Schritt
(E) Einspeisung von 4-Vinylcyclohexen in eine weitere Dehydrierzone und katalytische Dehydrierung zu Ethylbenzol oder Oxidehydrierung in Gegenwart von Sauerstoff zu Styrol.

Die Dehydrierung von 4-Vinylcyclohexen zu Ethylbenzol kann, wie in WO 94/01385 beschrieben, in der Gasphase an Magnesiumoxid, Zinkoxid, Calciumoxid, Strontiumoxid oder Bariumoxid als Katalysator durchgeführt werden. Die Dehydrierung kann in einer Vielzahl von geeigneten Reaktoren durchgeführt werden, wie kontinuierlich betriebenen Festbett- oder Wirbelbettreaktoren. Die Reaktionstemperatur beträgt im allgemeinen von 400 bis 625 °C, bevorzugt von 450 bis 600 °C, der Reaktionsdruck beträgt im allgemeinen von 1 bis 25 bar, vorzugsweise von 1 bis 10 bar.

Die Dehydrierung von 4-Vinylcyclohexen zu Ethylbenzol kann auch, wie in US 3,903,185 beschrieben, bei einer Temperatur von 350 bis 450°C und einem Druck von 2,5 bis 30 bar in Gegenwart von Wasserstoff an einem Katalysator, der ein oder mehrere Elemente der VI. bis VIII. Nebengruppe oder deren Oxide auf einem Aluminiumoxid-Träger enthält, durchgeführt werden. Bevorzugte Katalysatoren sind Rhenium, Palladium und/oder Platin auf Aluminiumoxid und Kobaltoxid/Molybdänoxid auf Aluminiumoxid.

Die Dehydrierung von 4-Vinylcyclohexen zu Ethylbenzol kann weiterhin, wie in US 4,029,715 beschrieben, bei 400 °C und Atmosphärendruck in Gegenwart eines Inertgases wie Wasserdampf oder Stickstoff an Kobaltmolybdat/Kaliumoxid auf Aluminiumoxid als Katalysator durchgeführt werden.

Die Dehydrierung von 4-Vinylcyclohexen zu Ethylbenzol kann ferner in der Gasphase bei Atmosphärendruck und Temperaturen um 300 °C an Palladium auf Magnesiumoxid als Katalysator durchgeführt werden.

Bei der Dehydrierung wird ein rohes Ethylbenzol erhalten, das als Nebenbestandteile nicht umgesetztes 4-Vinylcyclohexan und Ethylcyclohexan als Nebenprodukt enthalten kann.

Das bei der Butadien-Dimerisierung gebildete 4-Vinylcyclohexen kann auch in Gegenwart von Sauerstoff direkt zu Styrol dehydriert werden. Entsprechende Verfahren sind beispielsweise in US 3,502,736 und DE-A 2 612 082 beschrieben.

In einer Abwandlung des zuvor beschriebenen Verfahrens wird die Dehydrierung von 4-Vinylcyclohexen zu Styrol gemeinsam mit der n-Butan-Dehydrierung durchgeführt. Damit entfällt auch die Abtrennung von 4-Vinylcyclohexen aus dem Produktstrom der Dimerisierung.

Dieses Verfahren umfasst somit die Schritte
(A) Bereitstellung eines n-Butan enthaltenden Einsatzgasstromes,
(B') Einspeisung des n-Butan enthaltenden Einsatzgasstromes und eines 4-Vinylcyclohexen enthaltenden Gasstroms in eine Dehydrierzone und gemeinsame Dehydrierung in Gegenwart von Sauerstoff von n-Butan und 4-Vinylcyclohexen, wobei ein Styrol, Butadien, n-Butan, 1-Buten, 2-Buten, gegebenenfalls Ethylbenzol und weitere Nebenbestandteile enthaltender Produktstrom erhalten wird,
(C') Abtrennung von Styrol und gegebenenfalls Ethylbenzol und weiterer hochsiedender Nebenbestandteile aus dem Produktstrom der Dehydrierung,
(D') Einspeisung des Butadien, n-Butan, 1-Buten, 2-Buten enthaltenden Stroms in eine Dimerisierungszone und katalytische Dimerisierung von Butadien, wobei ein 4-Vinylcyclohexen, n-Butan, 1-Buten, 2-Buten und gegebenenfalls nicht umgesetztes Butadien enthaltender Produktstrom erhalten wird,
(E') Gewinnung des 4-Vinylcyclohexen enthaltenden Gasstroms aus dem Produktstrom der Dimerisierung und Einspeisung in die Dehydrierzone.

Weitere hochsiedende Nebenbestandteile, die bei der Dehydrierung gebildet werden können und gemeinsam mit Styrol und gegebenenfalls Ethylbenzol abgetrennt werden, sind Xylole, Toluol und Benzol.

Für die gemeinsame Dehydrierung von n-Butan und 4-Vinylcyclohexen in Gegenwart von Sauerstoff sind diejenigen der zuvor beschriebenen Dehydrierkatalysatoren geeignet, die ein Edelmetall der VIII. Nebengruppe, bevorzugt Platin und/oder Palladium, auf einem Träger enthalten. Daneben können die Dehydrierungskatalysatoren ein oder mehrere Elemente der I. und/oder II. Hauptgruppe, bevorzugt Kalium und/oder Cäsium, ein oder mehrere Elemente der III. Nebengruppe einschließlich der Lanthaniden und Actiniden, bevorzugt Lanthan und/oder Cer, ein oder mehrere Elemente der III. und/oder IV. Hauptgruppe aufweisen, bevorzugt Bor, Gallium, Silizium, Germanium, Zinn und/oder Blei, besonders bevorzugt Zinn enthalten.

Bevorzugte Ausführungsformen der erfindungsgemäßen Verfahren werden nachstehend mit Bezugnahme auf die Zeichnungen näher erläutert.

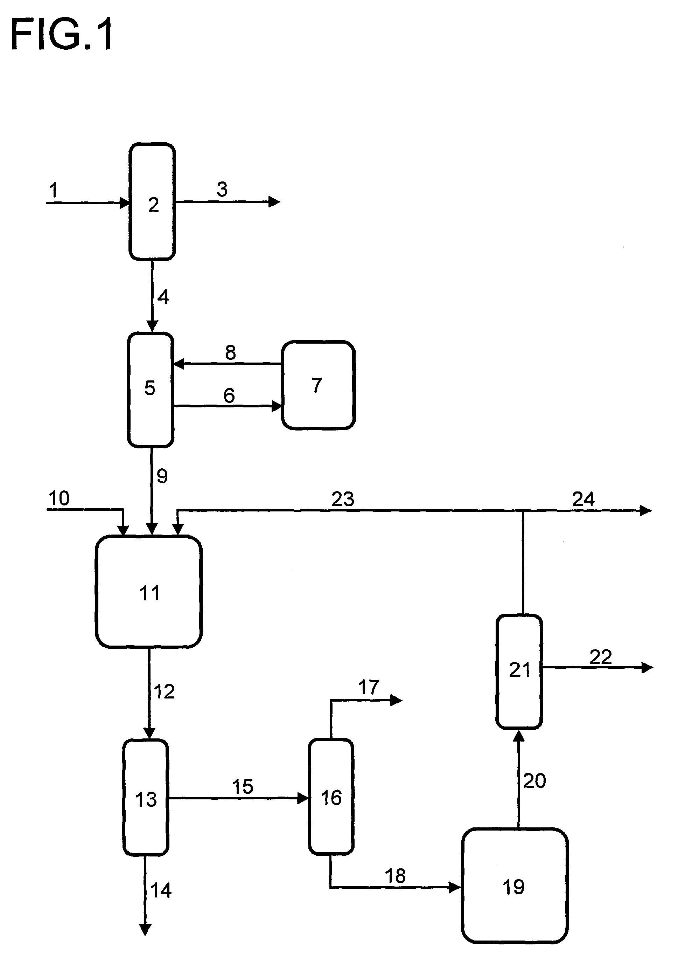

Figur 1 zeigt das Verfahrensfließbild einer bevorzugten Ausführungsform des erfindungsgemäßen Verfahrens. Ein Einsatzstrom 1 aus liquefied petroleum gas (LPG), das im wesentlichen aus Propan, n-Butan und Isobutan besteht, wird einer Rektifizierkolonne 2 zugeführt und in einen Strom 3 aus im wesentlichen Propan und gegebenenfalls Methan und Ethan und einen Strom 4 aus im wesentlichen n-Butan und Isobutan aufgetrennt. In der Rektifizierkolonne 5 wird das Butan-Gemisch in Isobutan 6 und n-Butan 9 aufgetrennt, wobei Isobutan in dem Isomerisierungsreaktor 7 zu einem n-Butan/Isobutan-Gemisch 8 isomerisiert wird, das in die Rektifizierkolonne 5 zurückgespeist wird. n-Butan wird als Einsatzgasstrom 9 in den Dehydrierreaktor 11 eingespeist, der vorzugsweise unter autothermalen Bedingungen unter Zuspeisung von Sauerstoff oder Luft als Co-Feed 10 betrieben wird. Der den Dehydrierreaktor verlassende Produktgasstrom 12, der neben Butadien, 1-Buten, 2-Buten und nicht umgesetztem n-Butan Nebenprodukte wie Wasserstoff, Kohlenstoffoxide, Stickstoff, Wasserdampf, Methan, Ethan, Ethen, Propan und/oder Propen enthält, wird nach Vorkühlung in Wärmetauschern in der Kühl- und Kondensationseinheit 13, beispielsweise einem Wasserrieselbett oder einem Fallfilmkondensator, soweit abgekühlt, dass Wasser und hochsiedende organische Nebenprodukte auskondensieren, die als Strom 14 aus dem Verfahren ausgeschleust werden. Die nicht auskondensierten Produktgasbestandteile werden als Strom 15 der Trennstufe 16 zugeführt, in der eine Abtrennung von Leichtsiedern und nicht kondensierbaren Nebenbestandteilen 17 (eine oder bevorzugt mehrere Komponenten aus der Gruppe Wasserstoff, Kohlenstoffoxide, Stickstoff, Methan, Ethan, Ethen, Propan und Propen) stattfindet. Die Trennstufe 16 kann als Rektifizierkolonne oder als Absorptions/Desorptionseinheit ausgestaltet sein. Der die C₄-Produkte der Dehydrierung und nicht umgesetztes n-Butan enthaltende Strom 18 wird dem Dimerisierungsreaktor 19 zugeführt, der einstufig oder mehrstufig sein kann. Der den Dimerisierungsreaktor verlassende Produktstrom 20 wird in der Rektifizierkolonne 21 in einen Strom 22 aus rohem 4-Vinylcyclohexen 22 und einen n-Butan, 1-Buten, 2-Buten und gegebenenfalls nicht umgesetztes Butadien enthaltenden Strom 23 aufgetrennt. Dieser wird in den Dehydrierreaktor 11 zurückgeführt. Optional kann ein Teilstrom 24 abgetrennt werden, der in auf Buten basierenden Verfahren wie der Maleinsäureanhydrid-Darstellung, der Oxo-Synthese, der Butendimerisierung, -trimerisierung und -metathese eingesetzt werden kann.

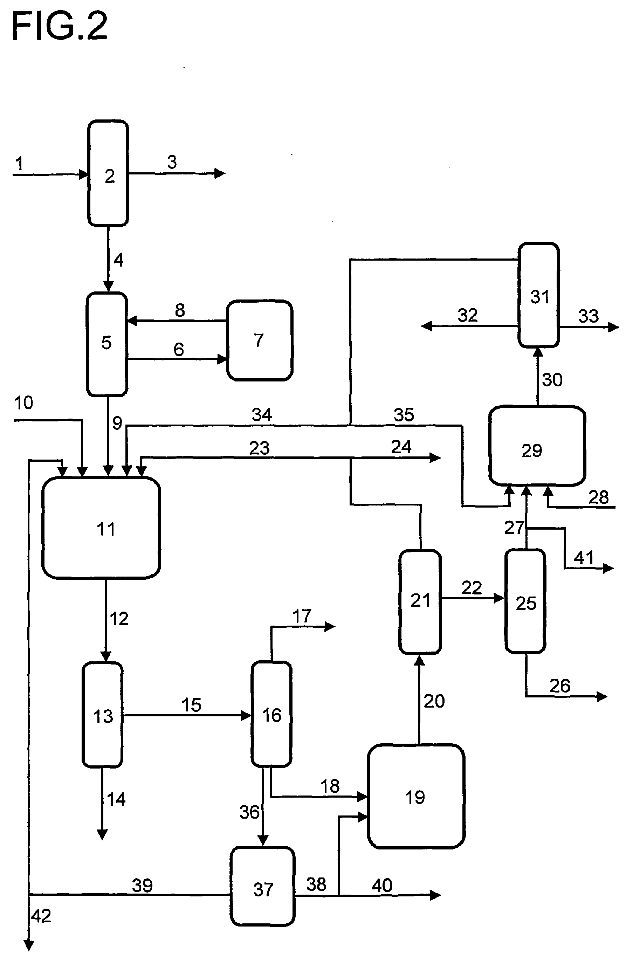

Figur 2 zeigt das Verfahrensfließbild einer weiteren bevorzugten Ausführungsform des erfindungsgemäßen Verfahrens. Dieses Verfahren unterscheidet sich von dem in Figur 1 dargestellten Verfahren im wesentlichen dadurch, dass der Butadien-Dimerisierung die Dehydrierung des gebildeten 4-Vinylcyclohexens folgt. Der in der Rektifizierkolonne 21 gewonnene Strom 22 aus rohem 4-Vinylcyclohexen wird optional in der Rektifizierkolonne 25 einer weiteren Aufreinigung unterworfen, wobei ein Strom aus Schwersiedern 26 abgetrennt wird. Der Strom 27 aus aufgereinigtem 4-Vinylcyclohexen wird dem Dehydrierreaktor 29 zugeführt, wo in Gegenwart von Wasserstoff 35 und Wasserdampf 28 die Dehydrierung zu Ethylbenzol stattfindet. Optional kann ein Seitenstrom 41 aus 4-Vinylcylohexen abgezogen und der katalytischen Oxidehydrierung zu Styrol oder einer anderen Verwendung zugeführt werden. Der Produktstrom der 4-Vinylcyclohexen-Dehydrierung wird in der Kühl- und Kondensationseinheit 31 mit Wasser abgekühlt, wobei ein Strom 32 aus wässriger Phase und ein Strom 33 aus rohem Ethylbenzol anfällt. Der bei der 4-Vinylcyclohexen-Dehydrierung gebildete Wasserstoff, der mit CO, CO₂, Methan, Ethan und Stickstoff verunreinigt sein kann ("Dehydrier-Wasserstoff'), kann zum Teil als Teilstrom 35 in den Dehydrierreaktor 29 zurückgeführt und zum Teil als Teilstrom 34 in den Dehydrierreaktor 11 zurückgeführt werden. Optional kann ein Teilstrom 36 der in der Trennstufe 16 abgetrennten C₄-Produkte einer weiteren Trennstufe 37 zugeführt werden und dort in einen Strom 39 aus 1-Buten, 2-Buten und n-Butan und einen Strom 38 aus Butadien aufgetrennt werden. Von dem Strom 38 kann ein Teilstrom 40 des Butadiens einer anderen Verwendung zugeführt werden. Der Strom 39 kann zumindest teilweise in den Dehydrierreaktor zurückgeführt werden, wobei ein Teilstrom 42 einer anderen Verwendung zugeführt werden kann.

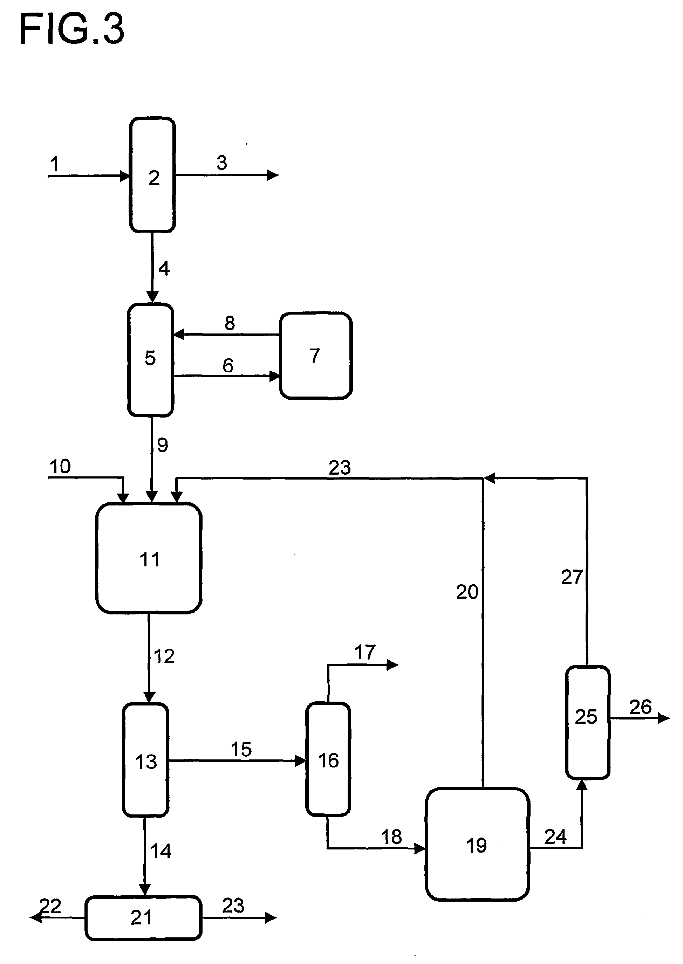

Figur 3 zeigt das Verfahrensfließbild einer weiteren Ausführungsform des erfindungsgemäßen Verfahrens. Dieses Verfahren unterscheidet sich von dem in Figur 1 dargestellten Verfahren im wesentlichen dadurch, dass der den Dimerisierungsreaktor verlassende Produktstrom 20 aus 4-Vinylcyclohexen, n-Butan, 1-Buten, 2-Buten und gegebenenfalls nicht umgesetztem Butadien in den Dehydrierreaktor 11 eingespeist wird, in dem gemeinsam n-Butan zu Butadien und 4-Vinylcyclohexen zu Styrol dehydriert werden. Entsprechend werden in der Kühl- und Kondensationseinheit 13 Wasserdampf und Styroldampf aus dem Produktgasgemisch 12 der Dehydrierung auskondensiert, als wässrig/organisches Gemisch 14 dem Phasenseparator 21 zugeführt und dort in eine wässrige Phase 22 und eine organische Phase 23 aus rohem Styrol aufgetrennt. Optional kann ein Teil des in dem Dimerisierungsreaktor 19 erhaltenen Produktgemischs als Strom 24 einer Rektifizierkolonne 25 zugeführt werden, wo ein Strom 26 aus C₄-Kohlenwasserstoffen abgetrennt und einer anderen Verwendung zugeführt werden kann. Der verbleibende Strom 27 aus 4-Vinylcyclohexen wird dem Dehydrierreaktor 11 zugeführt.

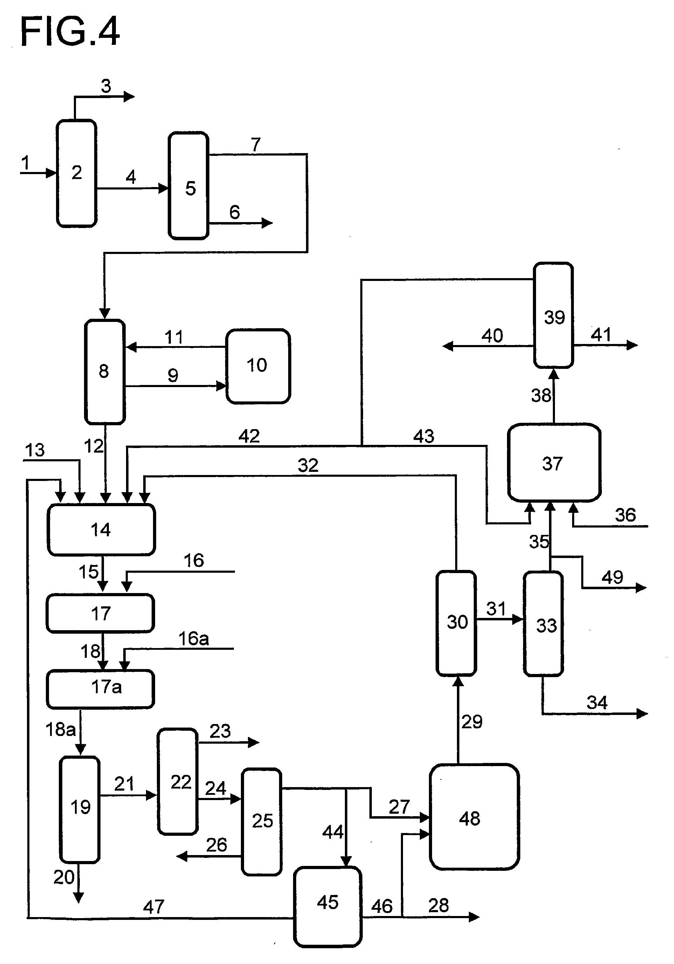

Figur 4 zeigt das Verfahrensfließbild einer besonders bevorzugten Ausführungsform des erfindungsgemäßen Verfahren. Ein Einsatzstrom 1 aus liquefied petroleum gas (LPG), das im wesentlichen aus Propan, n-Butan und Isobutan besteht und daneben auch Methan, Ethan oder Pentane enthalten kann, wird einer Rektifizierkolonne 2 zugeführt und in einen Strom 3 aus im wesentlichen Propan und gegebenenfalls Methan und Ethan und einen Strom 4 aus im wesentlichen n-Butan und Isobutan und gegebenenfalls Pentanen aufgetrennt. In der Rektifizierkolonne 5 werden gegebenenfalls Pentane 6 abgetrennt. Das Butan-Gemisch 7 wird in der Rektifizierkolonne 8 in Isobutan 9 und n-Butan 12 aufgetrennt, wobei Isobutan in dem Isomerisierungsreaktor 10 zu einem n-Butan/Isobutan-Gemisch 11 isomerisiert wird, das in die Rektifizierkolonne 8 zurückgespeist wird. n-Butan wird als Einsatzgasstrom 12 in die erste Dehydrierstufe 14 eingespeist, in der eine nicht-oxidative katalytische Dehydrierung von Butan zu 1-Buten, 2-Buten und Butadien stattfindet. Diese wird vorzugsweise unter autothermalen Bedingungen unter Zuspeisung von Sauerstoff oder Luft als Co-Feed 13 durchgeführt. Vorzugsweise wird die erste Dehydrierstufe mit Rückvermischung im Wirbelbett oder mit Teilkreisgasführung durchgeführt, beispielsweise wie in der nicht-vorveröffentlichten deutschen Patentanmeldung P 102 11 275.4 beschrieben. Der die erste Dehydrierstufe verlassende Produktgasstrom 15, der neben Butadien, 1-Buten, 2-Buten und nicht umgesetztem n-Butan Wasserdampf und Nebenbestandteile wie Wasserstoff, Kohlenstoffoxide, Stickstoff, Wasserdampf, Methan, Ethan, Ethen, Propan und/oder Propen enthält, wird einer zweiten Dehydrierstufe 17 zugeführt, in der unter Zuspeisung von Sauerstoff oder Luft als Co-Feed 16 eine oxidative Dehydrierung von 1-Buten und 2-Buten zu Butadien stattfindet. Die zweite Dehydrierstufe wird vorzugsweise in einem Rohrbündelreaktor durchgeführt. Die zweite Dehydrierstufe kann auch selbst mehrstufig, beispielsweise zweistufig, durchgeführt werden. Bei zweistufiger Ausführung der oxidativen Dehydrierung besteht die zweite Dehydrierstufe aus einer ersten oxidativen Dehydrierstufe 17 und einer zweiten oxidativen Dehydrierstufe 17a, wobei jeweils Luft oder Sauerstoff als Co-Feed 16 bzw. 16a zugespeist wird. Der die zweite Dehydrierstufe verlassende Produktgasstrom 18a (bei einstufiger Ausführung der oxidativen Dehydrierung ist dies der Produktgasstrom 18) enthält neben Butadien und nicht umgesetztem n-Butan Wasserdampf und Nebenbestandteile wie Wasserstoff, Kohlenstoffoxide, Stickstoff, Methan, Ethan, Ethen, Propan und/oder Propen, gegebenenfalls geringe Restmengen 1-Buten und 2-Buten und gegebenenfalls Sauerstoff und sauerstoffhaltige Kohlenwasserstoffe (Oxygenate). Der Produktgasstrom 18a wird, gegebenenfalls nach Vorkühlung in Wärmetauschern, in der Kühl- und Kondensationseinheit 19, die beispielsweise als Wasserrieselbett oder als Fallfilmkondensator ausgebildet sein kann, soweit abgekühlt, dass Wasser und hochsiedende organische Nebenprodukte wie hochsiedende Kohlenwasserstoffe und Oxygenate auskondensieren, die als Strom 20 aus dem Verfahren ausgeschleust werden. Die nicht auskondensierten Produktgasbestandteile werden als Strom 21 der Trennstufe 22 zugeführt, in der eine Abtrennung von Leichtsiedern und nicht kondensierbaren Nebenbestandteilen 23 (sofern im Produktgasstrom 18 vorhanden: Wasserstoff, Kohlenstoffoxide, Stickstoff, Methan, Ethan, Ethen, Propan, Propen und Sauerstoff) stattfindet. Die Trennstufe 22 kann als Rektifizierkolonne oder als Absorptions/Desorptionseinheit ausgestaltet sein. Der die C₄-Produkte der Dehydrierung, nicht umgesetztes n-Butan und gegebenenfalls Oxygenate wie Furan und Maleinsäureanhydrid enthaltende Strom 24 wird optional einer weiteren Trennstufe 25 zugeführt, die als Rektifizierkolonne oder Absorptions-/Desorptionseinheit ausgestaltet sein kann. In der Trennstufe 25 erfolgt eine Abtrennung von Oxygenaten und gegebenenfalls von verbliebenen Wasserspuren, die als Strom 26 aus dem Verfahren ausgeschleust werden. Der Strom 27 aus Butadien und n-Butan, der noch geringe Anteile an 1-Buten und 2-Buten enthalten kann, wird dem Dimerisierungsreaktor 28 zugeführt, der einstufig oder mehrstufig ausgeführt sein kann. Der den Dimerisierungsreaktor verlassende Produktstrom 29, der neben n-Butan und 4-Vinylcyclohexen noch nicht umgesetztes Butadien und geringe Anteile an 1-Buten und 2-Buten enthalten kann, wird in der Rektifizierkolonne 30 in einen Strom 31 aus rohem 4-Vinylcyclohexen und einen n-Butan und gegebenenfalls nicht umgesetztes Butadien, 1-Buten und 2-Buten enthaltenden Strom 32 aufgetrennt. Der Strom 32 wird in die (autotherme) Dehydrierstufe 14 zurückgeführt. Der in der Rektifizierkolonne 30 gewonnene Strom 31 aus rohem 4-Vinylcyclohexen wird optional in der Rektifizierkolonne 33 einer weiteren Aufreinigung unterworfen, wobei ein Strom 34 aus Schwersiedem abgetrennt wird. Der Strom 35 aus aufgereinigtem 4-Vinylcyclohexen wird dem Dehydrierreaktor 37 zugeführt, wo in Gegenwart von Wasserstoff 35 und unter Zuspeisung von Wasserdampf 36 die Dehydrierung zu Ethylbenzol stattfindet. Optional kann Wasserstoff 42 zugespeist werden. Optional kann ein Seitenstrom 49 aus 4-Vinylcylohexan abgezogen und der katalytischen Oxidehydrierung zu Styrol oder einer anderen Verwendung zugeführt werden. Der Produktstrom 38 der 4-Vinylcyclohexen-Dehydrierung wird in der Kühl- und Kondensationseinheit 39 mit Wasser abgekühlt, wobei ein Strom 41 aus wässriger Phase und ein Strom 40 aus rohem Ethylbenzol anfällt. Der bei der 4-Vinylcyclohexen-Dehydrierung gebildete Wasserstoff, der mit CO, CO₂, Methan, Ethan und Stickstoff verunreinigt sein kann ("Dehydrier-Wasserstoff"), kann zum Teil als Teilstrom 42 in die (autotherme) Dehydrierstufe 14 zurückgeführt und zum Teil als Teilstrom 43 in den Dehydrierreaktor 37 zurückgeführt werden. Optional kann ein Teilstrom 44 der in der Trennstufe 25 abgetrennten C₄-Produkte einer weiteren Trennstufe 45, beispielsweise einer Butadien-Wäsche (wie in Weissermehl/Arpe, Industrielle Organische Chemie, 5. Auflage 1998, S.120/121 beschrieben) zugeführt werden und dort in einen Strom 47 aus n-Butan und gegebenenfalls 1-Buten und 2-Buten und einen Strom 46 aus Butadien aufgetrennt werden. Von dem Strom 46 des Butadiens kann ein Teilstrom 48 abgetrennt und einer anderen Verwendung zugeführt werden, während der Reststrom dem Dimerisierungsreaktor 28 zugeführt wird.

## Patentansprüche

1. Verfahren zur Herstellung von 4-Vinylcylohexen mit den Schritten
(A) Bereitstellung eines n-Butan enthaltenden Einsatzgastroms,
(B) Einspeisung des n-Butan enthaltenden Einsatzgasstroms in mindestens eine Dehydrierzone und Dehydrierung von n-Butan zu Butadien, wobei ein Butadien, n-Butan, gegebenenfalls 1-Buten und 2-Buten und gegebenenfalls Wasserdampf und weitere Nebenbestandteile enthaltender Produktstrom erhalten wird,
(C) Einspeisung des Produktstroms der Dehydrierung, gegebenenfalls nach Abtrennung von Wasserdampf und Nebenbestandteilen, in eine Dimerisierungszone und katalytische Dimerisierung von Butadien, wobei ein 4-Vinylcyclohexen, n-Butan und gegebenenfalls 1-Buten, 2-Buten und nicht umgesetztes Butadien enthaltender Produktstrom erhalten wird,
(D) Abtrennung von 4-Vinylcyclohexen aus dem Produktstrom der Dimerisierung und Rückführung von n-Butan und gegebenenfalls 1-Buten, 2-Buten und nicht umgesetztem Butadien in die Dehydrierzone.

2. Verfahren nach Anspruch 1, **dadurch gekennzeichnet, dass** die Bereitstellung des n-Butan enthaltenden Dehydrier-Einsatzstromes die Schritt umfasst
(A1) Bereitstellung eines liquefied petroleum gas (LPG)-Stroms,
(A2) Abtrennung von Propan und gegebenenfalls Methan, Ethan und Pentanen aus dem LPG-Strom, wobei ein Butane enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

3. Verfahren nach Anspruch 1 oder 2, **dadurch gekennzeichnet, dass** die Dehydrierung von n-Butan zu Butadien als autotherme katalytische Dehydrierung durchgeführt wird.

4. Verfahren nach Anspruch 1 oder 2, **dadurch gekennzeichnet, dass** die Dehydrierung von n-Butan zu Butadien die Schritte umfasst
(B1) Einspeisung des n-Butan enthaltenden Einsatzgasstroms in eine erste Dehydrierzone und katalytische, nicht-oxidative Dehydrierung von n-Butan zu 1-Buten, 2-Buten und gegebenenfalls Butadien, wobei ein Butadien, n-Butan, 1-Buten, 2-Buten und gegebenenfalls Nebenbestandteile enthaltender Produktgasstrom erhalten wird,
(B2) Einspeisung des n-Butan, 1-Buten, 2-Buten, gegebenenfalls Butadien und gegebenenfalls Nebenbestandteile enthaltenden Produktgasstroms in eine zweite Dehydrierzone und oxidative Dehydrierung von 1-Buten und 2-Buten zu Butadien, wobei ein Butadien, n-Butan, Wasserdampf und gegebenenfalls Nebenbestandteile enthaltender Produktgasstrom erhalten wird.

5. Verfahren nach Anspruch 4, **dadurch gekennzeichnet, dass** die katalytische, nicht-oxidative Dehydrierung von n-Butan zu 1-Buten, 2-Buten und Butadien als autotherme Dehydrierung durchgeführt wird.

6. Verfahren nach einem der Ansprüche 1 bis 4, **dadurch gekennzeichnet, dass** vor der Dimerisierung eine Abtrennung von Wasserdampf und von Nebenbestandteilen, ausgewählt aus der Gruppe bestehend aus Wasserstoff, Kohlenmonoxid, Kohlendioxid, Stickstoff, Methan, Ethan, Ethen, Propan und Propen, aus dem Produktstrom der Dehydrierung durchgeführt wird.

7. Verfahren zur Herstellung von Ethylbenzol oder Styrol mit den Schritten (A), (B), (C) und (D), wie in einem der Ansprüche 1 bis 6 definiert, und dem zusätzlichen Schritt
(E) Einspeisung von 4-Vinylcyclohexen in eine weitere Dehydrierzone und katalytische Dehydrierung zu Ethylbenzol oder oxidative Dehydrierung in Gegenwart von Sauerstoff zu Styrol.

8. Verfahren zur Herstellung von Styrol mit den Schnitten
(A) Bereitstellung eines n-Butan enthaltenden Einsatzgasstromes,
(B') Einspeisung des n-Butan enthaltenden Einsatzgasstromes und eines 4-Vinylcyclohexen enthaltenden Gasstroms in eine Dehydrierzone und gemeinsame Dehydrierung in Gegenwart von Sauerstoff von n-Butan und 4-Vinylcyclohexen, wobei ein Styrol, Butadien, n-Butan, 1-Buten, 2-Buten, gegebenenfalls Ethylbenzol und weitere Nebenbestandteile enthaltender Produktstrom erhalten wird,
(C') Abtrennung von Styrol und gegebenenfalls Ethylbenzol und weiterer hochsiedender Nebenbestandteile aus dem Produktstrom der Dehydrierung,
(D') Einspeisung des Butadien, n-Butan, 1-Buten, 2-Buten enthaltenden Stroms in eine Dimerisierungszone und katalytische Dimerisierung von Butadien, wobei ein 4-Vinylcyclohexen, n-Butan, 1-Buten, 2-Buten und gegebenenfalls nicht umgesetztes Butadien enthaltender Produktstrom erhalten wird,
(E') Gewinnung des 4-Vinylcyclohexen enthaltenden Gasstroms aus dem Produktstrom der Dimerisierung und Einspeisung in die Dehydrierzone.

9. Verfahren nach Anspruch 8, **dadurch gekennzeichnet, dass** die gemeinsame Dehydrierung von n-Butan und 4-Vinylcyclohexen in Gegenwart eines Dehydrierkatalysators durchgeführt wird, der ein Edelmetall der VIII. Nebengruppe und daneben gegebenenfalls ein oder mehrere Elemente der I. und/oder der II. Hauptgruppe, ein oder mehrere Elemente der III. Nebengruppe einschließlich der Lanthaniden und Actiniden, ein oder mehrere Elemente der III. und/oder IV. Hauptgruppe, auf einem Träger enthält.

## Claims

1. A process for preparing 4-vinylcyclohexene, which comprises the steps
(A) providing an n-butane-containing feed gas stream,
(B) feeding the n-butane-containing feed gas stream into at least one dehydrogenation zone and dehydrogenating n-butane to butadiene to give a product stream comprising butadiene, n-butane, if appropriate 1-butene and 2-butene and if appropriate steam and further secondary constituents,
(C) feeding the product stream from dehydrogenation, if appropriate after separating off steam and secondary constituents, into a dimerization zone and catalytically dimerizing butadiene to give a product stream comprising 4-vinylcyclohexene, n-butane and if appropriate 1-butene, 2-butene and unreacted butadiene, and
(D) separating off 4-vinylcyclohexene from the product stream from the dimerization and recirculating n-butane and if appropriate 1-butene, 2-butene and unreacted butadiene to the dehydrogenation zone.

2. The process according to claim 1, wherein the provision of the n-butane-containing dehydrogenation feed stream comprises the steps
(A1) providing a liquefied petroleum gas (LPG) stream,
(A2) separating off propane and, if appropriate, methane, ethane and pentanes from the LPG stream to give a stream comprising butanes,
(A3) separating off isobutane from the stream comprising butanes to give the n-butane-containing feed gas stream and, if appropriate, isomerizing the isobutane which has been separated off to give an n-butane/isobutane mixture and recirculating the n-butane/isobutane mixture to the isobutane separation step.

3. The process according to claim 1 or 2, wherein the dehydrogenation of n-butane to butadiene is carried out as an autothermal catalytic dehydrogenation.

4. The process according to claim 1 or 2, wherein the dehydrogenation of n-butane to butadiene comprises the steps
(B1) feeding the n-butane-containing feed gas stream into a first dehydrogenation zone and catalytically, nonoxidatively dehydrogenating n-butane to 1-butene, 2-butene and if appropriate butadiene to give a product gas stream comprising butadiene, n-butane, 1-butene, 2-butene and if appropriate secondary constituents,
(B2) feeding the product gas stream comprising n-butane, 1-butene, 2-butene, if appropriate butadiene and if appropriate secondary constituents into a second dehydrogenation zone and oxidatively dehydrogenating 1-butene and 2-butene to butadiene to give a product gas stream comprising butadiene, n-butane, water vapor and if appropriate secondary constituents.

5. The process according to claim 4, wherein the catalytic nonoxidative dehydrogenation of n-butane to 1-butene, 2-butene and butadiene is carried out as an autothermal dehydrogenation.

6. The process according to any of claims 1 to 4, wherein steam and secondary constituents from the group consisting of hydrogen, carbon monoxide, carbon dioxide, nitrogen, methane, ethane, ethene, propane and propene are separated off from the product stream from the dehydrogenation prior to the dimerization.

7. A process for preparing ethylbenzene or styrene comprising the steps (A), (B), (C) and (D) as defined in any of claims 1 to 6 and the additional step
(E) feeding 4-vinylcyclohexene into a further dehydrogenation zone and catalytically dehydrogenating it to ethylbenzene or oxidatively dehydrogenating it in the presence of oxygen to give styrene.

8. A process for preparing styrene, which comprises the steps
(A) providing an n-butane-containing feed gas stream,
(B') feeding the n-butane-containing feed gas stream and a 4-vinylcyclohexene-containing gas stream into a dehydrogenation zone and jointly dehydrogenating n-butane and 4-vinylcyclohexene in the presence of oxygen to give a product stream comprising styrene, butadiene, n-butane, 1-butene, 2-butene, if appropriate ethylbenzene and further secondary constituents,
(C') separating off styrene and, if appropriate, ethylbenzene and further high-boiling secondary constituents from the product stream from the dehydrogenation,
(D') feeding the stream comprising butadiene, n-butane, 1-butene and 2-butene into a dimerization zone and catalytically dimerizing butadiene to give a product stream comprising 4-vinylcyclohexene, n-butane, 1-butene, 2-butene and if appropriate unreacted butadiene,
(E') isolating the 4-vinylcyclohexene-containing gas stream from the product stream from the dimerization and feeding it into the dehydrogenation zone.

9. The process according to claim 8, wherein the joint dehydrogenation of n-butane and 4-vinylcyclohexene is carried out in the presence of a dehydrogenation catalyst comprising a noble metal of transition group VIII together with, if appropriate, one or more elements of main groups I and/or II, one or more elements of transition group III including the lanthanides and actinides and/or one or more elements of main groups III and/or IV on a support.

## Revendications

1. Procédé pour la préparation de 4-vinylcyclohexène par les étapes
(A) de préparation d'un flux de gaz d'utilisation contenant du n-butane,
(B) d'injection du flux de gaz d'utilisation, contenant du n-butane, dans au moins une zone de déshydrogénation et déshydrogénation du n-butane en n-butadiène, avec obtention d'un flux de produits contenant du butadiène, du n-butane, éventuellement du 1-butène et du 2-butène et éventuellement de la vapeur d'eau et d'autres constituants secondaires,
(C) d'injection du flux de produits de la déshydrogénation, éventuellement après séparation de la vapeur d'eau et des constituants secondaires, dans une zone de dimérisation et dimérisation catalytique de butadiène, avec obtention d'un flux de produits contenant du 4-vinylcyclohexène, du n-butane et éventuellement du 1-butène, du 2-butène et du butadiène non transformé,
(D) de séparation du 4-vinylcyclohexène du flux de produits de la dimérisation et recyclage du n-butane et éventuellement du 1-butène, du 2-butène et du butadiène non transformés dans la zone de déshydrogénation.

2. Procédé selon la revendication 1, **caractérisé en ce que** la préparation du flux d'utilisation de déshydrogénation contenant du n-butane comprend les étapes
(A1) de préparation d'un flux de "liquefied petroleum gas" (LPG),
(A2) de séparation du propane et éventuellement du méthane, de l'éthane et des pentanes du flux de LPG, avec obtention d'un flux contenant des butanes,
(A3) de séparation de l'isobutane du flux contenant les butanes, avec obtention du flux de gaz d'utilisation contenant du n-butane, et éventuellement isomérisation de l'isobutane séparé en un mélange n-butane/isobutane et recyclage du mélange n-butane/isobutane dans la séparation de l'isobutane.

3. Procédé selon la revendication 1 ou 2, **caractérisé**
**en ce que** la déshydrogénation de n-butane en butadiène est réalisée sous forme de déshydrogénation catalytique autothermique.

4. Procédé selon la revendication 1 ou 2,
**caractérisé**
**en ce que** la déshydrogénation de n-butane en butadiène comprend les étapes
(B1) d'injection du flux de gaz d'utilisation, contenant du n-butane, dans une première zone de déshydrogénation et déshydrogénation catalytique, non oxydante du n-butane en 1-butène, 2-butène et éventuellement butadiène, avec obtention d'un flux gazeux de produits contenant du butadiène, du n-butane, du 1-butène, du 2-butène et éventuellement d'autres constituants secondaires,
(B2) d'injection du flux de gaz de produits, contenant du n-butane, du 1-butène, du 2-butène, éventuellement du butadiène et éventuellement des constituants secondaires, dans une deuxième zone de déshydrogénation et déshydrogénation oxydante du 1-butène et du 2-butène en butadiène, avec obtention d'un flux gazeux de produits contenant du butadiène, du n-butane, de la vapeur d'eau et éventuellement d'autres constituants secondaires.

5. Procédé selon la revendication 4, **caractérisé en ce que** la déshydrogénation catalytique, non oxydante de n-butane en 1-butène, 2-butène et butadiène est réalisée sous forme de déshydrogénation autothermique.

6. Procédé selon l'une quelconque des revendications 1 à 4, **caractérisé en ce qu'**on réalise, avant la dimérisation, une séparation de la vapeur d'eau et des constituants secondaires, choisis dans le groupe constitué par l'hydrogène, le monoxyde de carbone, le dioxyde de carbone, l'azote, le méthane, l'éthane, l'éthylène, le propane et le propylène, du flux de produits de la déshydrogénation.

7. Procédé pour la préparation d'éthylbenzène ou de styrène comprenant les étapes (A), (B), (C) et (D), telles que définies dans l'une quelconque des revendications 1 à 6, et l'étape supplémentaire
(E) d'injection de 4-vinylcyclohexène dans une autre zone de déshydrogénation et déshydrogénation catalytique en éthylbenzène ou déshydrogénation oxydante en présence d'oxygène en styrène.

8. Procédé pour la préparation de styrène par les étapes
(A) de préparation d'un flux de gaz d'utilisation contenant du n-butane,
(B') d'injection du flux de gaz d'utilisation contenant du n-butane et d'un flux gazeux contenant du 4-vinylcyclohexène dans une zone de déshydrogénation et déshydrogénation commune en présence d'oxygène du n-butane et du 4-vinylcyclohexène, avec obtention d'un flux de produits contenant du styrène, du butadiène, du n-butane, du 1-butène, du 2-butène, éventuellement de l'éthylbenzène et d'autres constituants secondaires,
(C') de séparation du styrène et éventuellement de l'éthylbenzène et d'autres constituants secondaires à point d'ébullition élevé du flux de produits de la déshydrogénation,
(D') d'injection du flux contenant du butadiène, du n-butane, du 1-butène, du 2-butène dans une zone de dimérisation et dimérisation catalytique de butadiène, avec obtention d'un flux de produits contenant du 4-vinylcyclohexène, du n-butane, du 1-butène, du 2-butène et éventuellement du butadiène non transformé,
(E') d'obtention du flux gazeux contenant du 4-vinylcyclohexène du flux de produits de la dimérisation et d'injection dans la zone de déshydrogénation.

9. Procédé selon la revendication 8, **caractérisé en ce que** la déshydrogénation commune de n-butane et de 4-vinylcyclohexène est réalisée en présence d'un catalyseur de déshydrogénation, qui contient un métal noble du VIIIème groupe secondaire et en outre éventuellement un ou plusieurs éléments du Ier et/ou du IIème groupe principal, un ou plusieurs éléments du IIIème groupe secondaire, comprenant les lanthanides et les actinides, un ou plusieurs éléments du IIIème et/ou du IVème groupe principal, sur un support.
